Europäisches Patentamt

European Patent Office

Office européen des brevets

(19)

(11) Veröffentlichungsnummer: **0 022 880**
**B1**

(12) # EUROPÄISCHE PATENTSCHRIFT

(45) Veröffentlichungstag der Patentschrift:
21.04.82

(21) Anmeldenummer: 79102545.5

(22) Anmeldetag: 19.07.79

(51) Int. Cl.³: **C 07 C 99/12**

(54) **Verfahren zur Trennung von Leucin, Isoleucin und Valin.**

(43) Veröffentlichungstag der Anmeldung:
**28.01.81 Patentblatt 81/4**

(45) Bekanntmachung des Hinweises auf die Patenterteilung:
**21.04.82 Patentblatt 82/16**

(84) Benannte Vertragsstaaten:
**BE CH DE FR GB NL**

(56) Entgegenhaltungen:
**DE-A1-2 417 375**
**Chemical Abstracts Band 48, Nr. 19, 10. Oktober 1954, Columbus, Ohio, USA**
**Y. YSUCHIYA »Separation of leucine-isoleucine mixture« Spalte 11737b**

**Chemical Abstracts Band 59, Nr. 8, 14. Oktober 1963, Columbus, Ohio, USA**
**K. Hayashi et al.: »Separatory purification of L-isoleucine and L-leucine« Spalte 10237g**

**Chemical Abstracts Band 81, Nr. 25, 23. Dezember 1974, Columbus, Ohio, USA**
**H. HAMAGUCHI »Removal of L-tyrosine from mixtures of L-isoleucine, L-leucine, and L-tyrosine« Seite 577, Spalte 2, Abstract Nr. 169839p**

(73) Patentinhaber: **MAGGI A.G., CH-8310 Kempttal (CH)**
(84) Benannte Vertragsstaaten: **CH DE**

(73) Patentinhaber: **SOCIETE DES PRODUITS NESTLE S.A., Case postale 353, CH-1800 Vevey (CH)**
(84) Benannte Vertragsstaaten: **BE FR GB NL**

(72) Erfinder: **Steinmetzer, Walter, Im Bodetal 9, D-3334 Süpplingen (DE)**

(74) Vertreter: **Wavre, Claude-Alain, 55, avenue Nestlé, CH-1800 Vevey (CH)**

## Verfahren zur Trennung von Leucin, Isoleucin und Valin

Die Erfindung betrifft ein Verfahren zur Trennung von Leucin, Isoleucin und Valin.

Die Aminosäuren Leucin, Isoleucin und Valin haben als gemeinsames Strukturmerkmal verzweigte aliphatische Seitenketten. Bedingt durch diese ähnlichen Strukturmerkmale zeigen diese Aminosäuren im physikalischen und chemischen Verhalten sehr ähnliche Eigenschaften, und die Trennung bereitet große Schwierigkeiten.

Die erste Trennung von Leucin und Isoleucin wurde über die Kupferkomplexe durchgeführt. Bei diesem Verfahren werden die getrockneten Kupferkomplexe der Aminosäuren mit Methanol extrahiert und dabei gehen die Kupfersalze von Isoleucin und Valin in Lösung. Die weitere Trennung von Valin und Isoleucin erfolgt auf einem sehr komplizierten Weg nach einer alkalischen Racemisierung. In ähnlichen Verfahren werden auch die Kobaltkomplexe der Aminosäuren durch Extraktion mit Alkohol aufgetrennt. Neben der schwierigen Trennung von Valin und Isoleucin ergeben sich bei diesen Verfahren noch Probleme bei der Rückgewinnung der Metalle und der weiteren Reinigung der Aminosäuren.

In anderen Verfahren wird die Fällung von Leucin mit aromatischen Sulfonsäuren beschrieben. So wird die Verwendung von 2-Bromtuol-5-sulfonsäure und Naphthalin-2-sulfonsäure für die Fällung von Leucin und 1-Chlor-4-naphthalinsulfonsäure oder 2-Naphthol-6-sulfonsäure für die Fällung von Isoleucin vorgeschlagen. Bei diesen Verfahren müssen durch zahlreiche Umkristallisationen die Präzipitate gereinigt werden und ein besonderes Problem bedeutet die Abtrennung der oft stark giftigen Fällungsmittel und des Valins, welches gemeinsam mit dem Isoleucin auskristallisiert.

Der vorliegenden Erfindung lag die Aufgabe zugrunde, ein neues einfaches Verfahren zur Trennung der Aminosäuren Leucin, Isoleucin und Valin ohne Verwendung von giftigen oder aggressiven Fällungsmitteln zu schaffen.

Das erfindungsgemäße Verfahren besteht aus einer stufenweisen Anreicherung von Isoleucin und Valin in den Mutterlaugen bei einer Leucin-Kristallisation im pH-Bereich von 1,5−2,0 und einer Umkehrung dieses Verhältnisses durch Kristallisation von Isoleucinhydrochlorid aus konzentrierter, vorzugsweise 20−30%iger Salzsäure und Anreicherung von Leucin in den Mutterlaugen.

Durch diese nach umfangreichen Untersuchungen gefundene Umkehrung des Löslichkeitsverhaltens von Leucin, Isoleucin und Valin in den zwei verschiedenen Systemen wird durch eine einfache fraktionierte Kristallisation eine Trennung dieser Aminosäuren erzielt. Zur Herstellung der reinen Aminosäuren können die in Wasser gelösten Aminosäurehydrochloride mit einem schwach basischen Anionenaustauscher behandelt und dabei die Salzsäure entfernt werden. Beim Konzentrieren der neutralen Lösungen kristallisieren dann die reinen Aminosäuren aus.

Als Ausgangsmaterial zur Durchführung dieses neuen Trennungsganges kann beinahe jede in der Gewinnung von Aminosäuren aus den verschiedensten Proteinquellen, wie z. B. entzukkerten Melassen, Getreide- oder Maiskeimen, Öltrester, Mikroorganismen, insbesondere Hefen oder Kasein praktisch vorkommende, hauptsächlich Leucin, Isoleucin und Valin enthaltende Lösung verwendet werden. Bevorzugtes Ausgangsmaterial ist eine in der deutschen Patentanmeldung 2 906 034 beschriebene Aminosäurelösung, welche durch Behandlung über einen stark basischen Anionenaustauscher von Phenylalanin und Tyrosin befreit wurde.

Die betreffende Lösung kann mit Salzsäure auf einen pH-Wert von 1,5−2,0 eingestellt und konzentriert werden. Beim Konzentrieren kristallisiert ein an Leucin angereicherter Bodenkörper aus, während sich Isoleucin mit Valin in den Mutterlaugen anreichert. Der leucinreiche Bodenkörper kann abgetrennt werden, und durch erneutes Konzentrieren der Mutterlauge im pH-Bereich 1,0−2,0 kann wiederum ein leucinreicher Bodenkörper erhalten werden, während die Mutterlauge noch stärker an Isoleucin und Valin angereichert ist. Der in der zweiten Stufe anfallende leucinreiche Bodenkörper kann in die vorhergehende Kristallisationsstufe zurückgeführt oder in Wasser gelöst werden um bei einem pH-Wert von 1,5−2,0 eine reinere Leucinfraktion auszukristallisieren. Durch Wiederholung dieses Vorganges kann reines L-Leucin als Bodenkörper gewonnen werden. Die an Isoleucin und Valin angereicherten Mutterlaugen können mit gleichen Teilen konzentrierter Salzsäure versetzt und konzentriert werden. Der Salzsäureanteil kann so hoch bemessen sein, daß beim Konzentrieren zum Schluß die azeotrope Mischung Salzsäure/Wasser, d. h., 21%ige Salzsäure, erhalten wird. Beim Abkühlen der salzsauren Lösung kristallisiert als Bodenkörper ein angereichertes Isoleucinhydrochlorid aus, während sich Valin und Leucinhydrochlorid in der Mutterlauge anreichern. Durch Wiederholung des Kristallisationsprozesses, d. h. Lösen des Isoleucinhydrochlorids in 20−30%iger Salzsäure bei 60−70°C und Auskristallisation, kann das Isoleucinhydrochlorid weiter gereinigt werden.

Vorzugsweise werden diese verschiedenen Kristallisationen unter Rückführung von Mutterlaugen oder Bodenkörper so oft wiederholt bis die gewünschte Reinheit von Leucin und Isoleucin erreicht ist.

Aus den Aminosäurehydrochloriden werden vorzugsweise durch Behandlung mit schwach basischen Anionenaustauscher die freien Aminosäuren gewonnen.

In der Mutterlauge der Isoleucinhydrochlorid-Kristallisation reichert sich das Valin an. Die Mutterlauge kann konzentriert und nach Zusatz von Butanol oder Isobutanol kann eine Veresterung der Aminosäuren vorgenommen werden. Während dieser Veresterung kann das Wasser durch Sieden am Rückfluß azeotrop abdestilliert und damit eine sehr hohe Ausbeute an Aminosäureester erzielt werden. Nach been-deter Veresterung kann die Aminosäureesterhy-drochloride mit Natronlauge neutralisiert und nach Abtrennung der wäßrigen Phase die im Butanol gelösten Aminosäureester durch fraktionierte Destillation getrennt werden. So kann der Valin-Isobutylester bei einem Druck von 3 mbar bei 65°C abdestilliert werden, während die Butylester des Leucins und Isoleucins als Destillationsrückstand erhalten werden. Die Aminosäureester können dann mit 6n Salzsäure durch Kochen am Rückfluß hydrolysiert, und durch Einengen der salzsauren Lösung die jeweiligen Hydrochloride der Aminosäuren auskristallisiert werden. Die aus dem Destillations-rückstand gewonnenen Hydrochloride des Leu-cins und Isoleucins können in den oben beschriebenen Trennungsgang zurückgeführt werden, während das Valinhydrochlorid in Wasser gelöst und mit einem schwach basi-schen Anionenaustauscher die Salzsäure abge-trennt werden kann. Beim Konzentrieren der neutralen Lösung kristallisiert reines Valin aus.

Die Erfindung wird anhand der folgenden Beispiele näher erläutert. Die Prozentangaben sind in Gewicht ausgedrückt.

Beispiel 1

Ausgehend von einer Lösung mit 0,7 Teilen Isoleucin sowie 0,17 Teilen Valin auf 1 Teil Leucin, wird durch Konzentrieren und Kristallisation bei pH 1,7 ein Bodenkörper A mit 0,3 und eine Mutterlauge A' mit 1,1 Teilen Isoleucin bezogen auf 1 Teil Leucin gewonnen. Durch weiteres Konzentrieren der Mutterlauge A' bei pH 1,7 wird dann ein Bodenkörper B mit 0,5 Teilen Isoleucin und eine Mutterlauge B' mit 2,5 Teilen Isoleucin erhalten. Diese Mutterlauge B' wird erneut konzentriert und kristallisiert und ein Bodenkörper C mit 1,0 Teilen Isoleucin und eine Mutterlauge C' mit 4,0 Teilen Isoleucin erhalten. Die Mutterlauge C' wird im Verhältnis 1:1 mit 30%iger Salzsäure versetzt und konzentriert. Beim Abkühlen kristallisiert ein Bodenkörper D mit 7,2 Teilen Isoleucin aus, und die Mutterlauge D' enthält 1,8 Teile Isoleucin bezogen auf Leucin, d. h., es findet eine Umkehrung der Löslichkeitsverhältnisse statt. Der Bodenkörper D wird in 6n Salzsäure bei 70°C gelöst, abgekühlt und es kristallisiert ein Bodenkörper E mit 24 Teilen Isoleucin aus, und die Mutterlauge E' enthält 2,7 Teile Isoleucin.

In der folgenden Tabelle sind die jeweiligen, in einer willkürlichen Gewichtseinheit ausgedrückten Inhalte der Bodenkörper A, B, C, D, E und der Mutterlaugen A', B', C', D', E' an Isoleucin, Leucin und Valin angegeben. Aus dieser Tabelle ist die Verteilung des Valins bezogen auf Isoleucin leicht ersichtlich. In den ersten Stufen A, B und C reichert sich das L-Valin gemeinsam mit dem Isoleucin in den Mutterlaugen an, und bei der Kristallisation aus 6n Salzsäure erfolgt dann die Trennung Isoleucin und Valin durch Anreicherung von Valin in der Mutterlauge und Anreicherung des Isoleucins in den Bodenkörpern der Stufen D und E.

| Stufe | Isoleucin | Leucin | Velin |
|---|---|---|---|
| Ausgangs-lösung | 100 | 140 | 24 |
| A | 20 | 67 | 1,5 |
| A' | 80 | 73 | 22,5 |
| B | 25 | 50 | 3,7 |
| B' | 55 | 22 | 19 |
| C | 10 | 10 | 2 |
| C' | 45 | 12 | 16 |
| D | 28 | 4 | 4,5 |
| D' | 17 | 9 | 12,5 |
| E | 19 | 0,8 | 0,5 |
| E' | 9 | 3,2 | 3,5 |

Beispiel 2

2000 ml einer Aminosäurelösung mit 90 g Leucin, 70 g Isoleucin und 20 g Valin werden mit Salzsäure auf einen pH-Wert von 1,9 eingestellt und im Vakuumverdampfer auf ca. 1000 ml konzentriert. Nach dem Abkühlen wird ein Bodenkörper mit 50 g Leucin, 10 g Isoleucin und 1 g Valin erhalten, während in der Mutterlauge 40 g Leucin, 60 g Isoleucin und 19 g Valin verbleiben. Die Mutterlauge wird nach Abtrennung der Leucinfraktion erneut konzentriert, und es wird wieder ein Leucinrückstand erhalten, welcher beim nächsten Zyklus am Anfang des Prozesses eingesetzt wird. Die Mutterlauge aus der zweiten Kristallisation enthält 20 g Leucin, 50 g Isoleucin und 17 g Valin. Nach Zusatz der gleichen Menge an 20%iger Salzsäure wird diese Mutterlauge im Vakuum konzentriert. Nach dem Abkühlen kristallisiert ein Isoleucinhydrochlorid mit einer Reinheit von 80% aus. Dieses Isoleucinhydrochlorid wird in der gleichen Menge 20%iger Salzsäure bei 70°C gelöst, abgekühlt und wiederum Isoleucinhydrochlorid auskristallisiert. Dieses Isoleucinhydrochlorid hat bereits eine Reinheit von 95 – 98%.

### Beispiel 3

Nach zwei Kristallisationsstufen bei pH 1,9 und zwei Kristallisationsstufen aus 20%iger Salzsäure, wird eine Mutterlauge aus der Isoleucinhydrochlorid-Fällung mit 10% Valin, 7% Isoleucin und 7% Leucin erhalten. Diese Mutterlauge wird im Verhältnis 1 : 1 mit Isobutanol versetzt und durch Kochen am Rückfluß unter ständiger Abtrennung des überdestillierten Wassers 24 Stunden lang verestert. Nach beendeter Veresterung wird die Lösung der Esterhydlochloride mit verdünnter Natronlauge neutralisiert und die wäßrige Phase abgetrennt. In der Butanolphase sind die freien Aminosäureisobutylester enthalten. Der überschüssige Butylalkohol wird im Vakuum abdestilliert und anschließend durch fraktionierte Destillation der Valinester vom Leucin- und Isoleucinester abgetrennt. Bei einem Vakuum von 3 mbar siedet der Isobutylester des Valins bei 65°C. Der abdestillierte Valinisobutylester wird mit der doppelten Menge 6n Salzsäure 3 Stunden am Rückfluß gekocht, anschließend im Vakuum konzentriert und das auskristallisierte reine Valinhydrochlorid abgetrennt.

### Beispiel 4

200 g eines in der im Beispiel 2 beschriebenen Weise erhaltenen Isoleucinhydrochlorids werden in 2 l Wasser gelöst und über eine Austauschersäule mit 1 l schwach basischen Anionenaustauscher geleitet. Der Durchlauf erfolgt mit einer Geschwindigkeit von 2 Bettvolumen pro Stunde. Anschließend wird die Säule mit Wasser nachgewaschen und der Auslauf mit Waschwasser im Vakuum konzentriert. Nach dem Abkühlen wird das auskristallisierte reine Isoleucin abgetrennt und getrocknet.

### Patentansprüche

1. Verfahren zur Trennung von Leucin, Isoleucin und Valin, dadurch gekennzeichnet, daß eine Lösung dieser drei Aminosäuren bei einem pH-Wert von 1,5 – 2,0 konzentriert und eine an Isoleucin und Valin angereicherte Mutterlauge und ein an Leucin angereicherter Bodenkörper gewonnen wird.

2. Verfahren nach Anspruch 1, dadurch gekennzeichnet, daß die an Isoleucin und Valin angereicherte Mutterlauge mit konzentrierter Salzsäure versetzt und nach dem Konzentrieren eine Valin und Leucin angereicherte Mutterlauge und ein an Isoleucin angereicherter Bodenkörper gewonnen wird.

3. Verfahren nach Anspruch 1 und 2, dadurch gekennzeichnet, daß diese verschiedenen Kristallisationen unter Rückführung von Mutterlaugen oder Bodenkörpern so oft wiederholt werden, bis die gewünschte Reinheit von Leucin und Isoleucin erreicht ist.

4. Verfahren nach Anspruch 1 bis 3, dadurch gekennzeichnet, daß aus den Aminosäurehydrochloriden durch Behandlung mit schwach basischen Anionenaustauschern die freien Aminosäuren gewonnen werden.

### Claims

1. A process for the separation of leucine, isoleucine and valine which comprises concentrating a solution of these three amino acids at a pH-value of from 1.5 to 2.0 to produce a first mother liquor, enriched with isoleucine and valine and a first solid phase, enriched with leucine, and separating the mother liquor from the solid phase.

2. A process according to claim 1 which further comprises adding hydrochloric acid to the mother liquor enriched with isoleucine and valine, concentrating the mother liquor to produce a second mother liquor, enriched with valine and leucine, and a second solid phase, enriched with isoleucine, and separating the second mother liquor from the second solid phase.

3. A process according to claim 2 in which prior to the addition of hydrochloric acid the mother liquor is concentrated at least twice, a solid phase being separated after each concentration step.

4. A process according to claim 2 or claim 3 which further comprises treating amino acid hydrochlorides formed upon addition of hydrochloric acid with a weakly basic ion exchanger and recovering free amino acids thus liberated.

### Revendications

1. Procédé de séparation de la leucine, l'isoleucine et la valine, caractérisé par le fait que l'on concentre une solution de ces 3 acides aminés à un pH de 1,5 à 2,0 et l'on obtient des eaux mères enrichies en isoleucine et valine et un sédiment enrichi en leucine.

2. Procédé selon la revendication 1, caractérisé par le fait que l'on ajoute de l'acide chlorhydrique concentré aux eaux mères enrichies en isoleucine et valine, on concentre et l'on obtient des eaux mères enrichies en valine et leucine et un sédiment enrichi en isoleucine.

3. Procédé selon les revendications 1 et 2, caractérisé par le fait que l'on réalise ces différentes cristallisations avec recyclage des eaux mères et de sédiments aussi souvent qu'il est nécessaire pour obtenir le degré de pureté désiré de la leucine et de l'isoleucine.

4. Procédé selon les revendications 1 à 3, caractérisé par le fait que l'on obtient des acides aminés libres à partir des chlorhydrates d'acides aminés par traitement sur une résine échangeuse d'anion faiblement basique.